# EUROPÄISCHE PATENTANMELDUNG

(11) **EP 2 228 076 A1**
(43) Veröffentlichungstag der Anmeldung: **15.09.2010**
(21) Anmeldenummer: 10152609.3
(22) Anmeldetag: 04.02.2010
(51) Int. Cl.: A61L 2/20

(54) **Verfahren und Vorrichtung zur Desinfektion eines Raums**

(30) Priorität: 24.02.2009 DE 102009001106
(71) Anmelder: caverion GmbH, 70499 Stuttgart (DE)
(72) Erfinder: Martinsteg, Hans, 52159 Roetgen (DE)
(74) Vertreter: Bauer, Dirk

(57) **Zusammenfassung**

Die Erfindung betrifft ein Verfahren sowie eine Vorrichtung (1) zur Desinfektion eines Raums (2), insbesondere zum Abtöten von pathogenen Mikroorganismen wie Bakterien, Viren, Pilzen und deren Sporen, wobei der Raum (2) vor Beginn des Desinfektionsvorgangs gasdicht abgeschlossen wird und in den Raum (2) ein Desinfektionsmittel eingebracht wird und im Wesentlichen an allen Orten in dem Raum (2) eine für die beabsichtigte Desinfektionswirkung hinreichend hohe Konzentration des Desinfektionsmittels in der Luft des Raums (2) erzeugt wird und wobei das Desinfektionsmittel während einer Zeitdauer auf die in dem Raum (2) befindlichen Mikroorganismen einwirkt. Damit der Zeitaufwand zur Aufbereitung des Raums für seine erneute Nutzung deutlich reduziert wird, ist es gemäß der Erfindung vorgesehen, dass die Luft des Raums (2) nach Ablauf der Zeitdauer so lange über oder durch ein Adsorptionsmaterial geführt und das Desinfektionsmittel an dem Adsorptionsmaterial adsorbiert wird, bis die Konzentration des Desinfektionsmittels in der Luft des Raums (2) unter einen Grenzwert abgesenkt worden ist.

## Beschreibung

Die vorliegende Erfindung betrifft ein Verfahren zur Desinfektion eines Raums, insbesondere zum Abtöten von pathogenen Mikroorganismen wie Bakterien, Viren, Pilzen und deren Sporen, wobei der Raum vor Beginn des Desinfektionsvorgangs gasdicht abgeschlossen wird und in den Raum ein Desinfektionsmittel eingebracht wird und im Wesentlichen an allen Orten in dem Raum eine für die beabsichtigte Desinfektionswirkung hinreichend hohe Konzentration des Desinfektionsmittels in der Luft des Raums erzeugt wird und wobei das Desinfektionsmittel während einer Zeitdauer auf die in dem Raum befindlichen Mikroorganismen einwirkt. Ferner hat die Erfindung eine Vorrichtung zur Desinfektion eines Raums nach dem Oberbegriff des Anspruchs 5 zum Gegenstand.

### Stand der Technik

Die Desinfektion eines Raums kann in vielen Fällen nötig sein, beispielsweise bei Laborräumen von Hochsicherheitslaboren, in denen mit den eingangs aufgezählten Mikroorganismen gearbeitet wird.

Gemäß den Technischen Regeln für Gefahrstoffe (TRGS), insbesondere den TRGS 522, erfolgt die Desinfektion eines Raums mittels eines in dem Raum positionierten Apparates, in dem Formalin "aufgekocht" wird, wodurch gasförmiges Formaldehyd freigesetzt wird. Vor Beginn der Desinfektion müssen sämtliche nach außen führenden Öffnungen des Raums abgedichtet sein. Die erforderliche freizusetzende Menge an Formaldehyd sowie die erforderliche Einwirkzeit richtet sich nach dem Raumvolumen. Die Desinfektion des Raums mit einem gasförmigen Desinfektionsmittel ist besonders effektiv, da das Gas an alle Stellen des Raums gelangen kann, selbst in eventuell vorhandene Fugen oder Ritzen, die auf andere Weise kaum zugänglich sind.

Nach Ablauf der Einwirkzeit des Desinfektionsmittels ist es zunächst erforderlich, die Luft des Raums zu neutralisieren, bevor dieser wieder nutzbar ist. Da der nach Ablauf der Einwirkzeit des Formaldehyds kontaminierte Raum jedoch vor seiner Neutralisation nicht geöffnet werden kann, müssen sich die Mittel zur Neutralisation ebenfalls bereits vor Beginn der Desinfektion in dem zu desinfizierenden Raum befinden. Zur Neutralisation wird typischerweise zu verdampfendes Ammoniak verwendet, das mit dem Formaldehyd zu dem ungiftigen Stoff Urotropin reagiert. Die Aktivierung der Mittel zur Freisetzung des Formaldehyds und des Ammoniaks, die aufeinander folgend eingeschaltet werden müssen, kann auf einfache Weise mittels einer Zeitschaltuhr erfolgen.

Nachteil der vorbeschriebenen Art der Desinfektion ist, dass sich das bei der Neutralisation entstehende Urotropin auf den gesamten Oberflächen des Raums niederschlägt und diese verschmutzen. Die Entfernung des Urotropin- Belags - die zur erneuten Nutzung des Raums meist unabdingbar, zumindest aber aus optischen Gründen angezeigt ist - ist nur manuell möglich und erfordert einen großen Arbeits- und somit Zeitaufwand.

Das vorbeschriebene Verfahren zur Desinfektion eines Raums ist sehr zeitaufwändig und setzt sich aus der Einwirkzeit des Formalins, der Neutralisationszeit und dem Zeitaufwand für die anschließende Reinigung zusammen.

### Aufgabe

Aufgabe der vorliegenden Erfindung ist es nun, die Desinfektion eines Raums nach der bekannten Art derart weiterzuentwickeln, dass der Zeitaufwand zur Aufbereitung des Raums für seine erneute Nutzung deutlich reduziert wird.

### Lösung

Verfahrensmäßig wird diese Aufgabe dadurch gelöst, dass die Luft des Raums nach Ablauf der Zeitdauer so lange über oder durch ein Adsorptionsmaterial geführt und das Desinfektionsmittel an dem Adsorptionsmaterial adsorbiert wird, bis die Konzentration des Desinfektionsmittels in der Luft des Raums unter einen Grenzwert abgesenkt worden ist.

Nachdem die Luft des Raums beispielsweise auf herkömmliche Weise mit dem Desinfektionsmittel versetzt wurde, muss der Raumluft folglich bei dem erfindungsgemäßen Verfahren kein dritter Stoff zugeführt werden, mittels dem eine Neutralisation herbeigeführt wird, vielmehr wird das zugesetzte Desinfektionsmittel wieder aus der Raumluft entfernt. Dabei ist es unerheblich, ob sich das Adsorptionsmaterial in dem Raum selbst befindet oder aber außerhalb des Raums angeordnet ist.

Beispielsweise kann die mit dem Desinfektionsmittel versetzte Luft des Raums durch geeignete Mittel angesaugt und über das Adsorptionsmaterial geführt werden, wobei die abgesaugte Luft durch frische Luft, die über einen entsprechenden Einlass im Raum nachströmen kann, ersetzt wird.

Alternativ kann die Führung über oder durch ein Adsorptionsmaterial, an dem das Desinfektionsmittel adsorbiert wird, über ein bereits vorhandenes Zuluft- und Abluftsystem des Raums erfolgen, wobei die Luft des Raums in einem geschlossenen Kreislauf solange im Umluftverfahren geleitet wird, bis sich nahezu das gesamte Desinfektionsmittel an dem Adsorptionsmaterial angelagert hat, das eine Filterfunktion ausübt. Nachdem die Konzentration des Desinfektionsmittels unter den festgelegten Grenzwert abgesenkt worden ist, ist der Raum wieder nutzbar. Das nunmehr belastete Adsorptionsmaterial kann auf einfache Weise vorschriftsmäßig entsorgt werden.

Da bei dem erfindungsgemäßen Verfahren auf eine Neutralisation im herkömmlichen Sinne verzichtet wird, entfällt der Zeitaufwand für die bisher erforderliche anschließende Reinigung der Oberflächen des Raums von dem Neutralisationsprodukt vollkommen, was sowohl zu einer Zeitersparnis und somit auch zu einer Kostenersparnis für den Desinfektionsvorgang führt.

Bei dem erfindungsgemäßen Verfahren ist es ferner möglich, das Desinfektionsmittel ebenfalls über das vorhandenes Zuluft- und Abluftsystem des Raums aufzugeben, worin ein weiterer Vorteil dahingehend besteht, dass ebenfalls die durchströmten Leitungen beziehungsweise Kanäle des Lüftungssystems desinfiziert werden. Vorzugsweise kann die Führung der Luft während der Einwirkzeit des Desinfektionsmittels auch in einem Luftkreislauf (Umluftverfahren) erfolgen, ohne über oder durch ein Absorptionsmaterial geführt zu werden. Die Führung der Luft des Raums über das Absorptionsmaterial erfolgt dann erst nachdem auch die durchströmten Lüftungsleitungen desinfiziert sind.

Vorteilhafterweise wird als Desinfektionsmittel Formaldehyd oder Formalin und als Adsorptionsmaterial Aktivkohle verwendet.

Gemäß einer Ausbildung des erfindungsgemäßen Verfahrens ist es vorgesehen, dass die Luft innerhalb des Raums in einem Kreislauf und dabei über oder durch das Adsorptionsmaterial geführt wird, wobei die Luft an einer ersten Stelle in dem Raum angesaugt und an einer zweiten Stelle in dem Raum wieder abgegeben wird. Wichtig hierbei ist, dass im Laufe der Einwirkzeit möglichst das gesamte Luftvolumen des Raumes durch das Desinfektionsmittel erfasst wird. Dieses Verfahren ist insbesondere dann vorteilhaft, wenn der zu desinfizierende Raum kein Lüftungssystem aufweist, das als Grundlage für einen geschlossenen Luftkreislauf dienen kann.

Es ist besonders vorteilhaft, wenn die Luft innerhalb des Raums vor und/oder während der Einbringung des Desinfektionsmittels, das heißt vor Beginn des eigentlichen Desinfektionsvorgangs, erwärmt wird. Durch die Erhöhung der Lufttemperatur innerhalb des Raumes wird gleichzeitig die Oberflächentemperatur der Innenflächen des Raumes und der in dem Raum befindlichen Einrichtungen erwärmt, so dass einem Kondensieren des im nächsten Schritt eingebrachten Desinfektionsmittels entgegengewirkt wird. Auch während der Einwirkzeit kann die Beheizung des Raums beibehalten werden, um einem Abkühlen und damit der Kondensationsgefahr entgegen zu wirken.

Vorrichtungsgemäß wird die eingangs gestellte Aufgabe durch eine Vorrichtung zur Desinfektion eines Raumes, insbesondere zum Abtöten von pathogenen Mikroorganismen wie Bakterien, Milben, Pilzen und deren Sporen, mit einem Vorrat eines Desinfektionsmittels und einer Abgabeeinrichtung, von der ein Strom des Desinfektionsmittels in die Luft des Raums abgebbar ist, gelöst, die gekennzeichnet ist durch eine Aufnahmeeinrichtung, von der ein Strom aus Luft und Desinfektionsmittel aus dem Raum in die Vorrichtung aufnehmbar ist und eine Adsorptionseinrichtung, die einen Vorrat eines Adsorptionsmaterials enthält, über oder durch das der von der Aufnahmeeinrichtung aufgenommene Strom leitbar ist, wodurch das Desinfektionsmittel aus dem Strom entfernbar und somit die Konzentration des Desinfektionsmittels in der Luft in dem Raum absenkbar ist. Eine derartige Vorrichtung ist insbesondere zur Durchführung des erfindungsgemäßen Verfahrens geeignet.

Um auf einfache Weise einen geschlossenen Luftkreislauf zu erhalten, in dem die Luft des Raumes im Umluftverfahren geführt wird, ist es vorteilhaft, wenn der über oder durch das Adsorptionsmaterial geleitete und in seiner Konzentration des Desinfektionsmittels reduzierte Strom von einer Rückführeinrichtung, die zumindest teilweise von der Abgabeeinrichtung gebildet ist, in den Raum zurückführbar ist. Dass die Rückführeinrichtung zumindest teilweise von der Abgabeeinrichtung gebildet wird, soll im vorliegenden Fall bedeuten, dass insbesondere Leitungen der Abgabeeinrichtung, Bestandteil des Rückführsystems sind.

Wenn der Raum ein Lüftungssystem aufweist, ist es gemäß einer Weiterbildung der Erfindung besonders vorteilhaft, wenn die Vorrichtung fest in einem Gebäude, das den Raum aufweist, installiert ist und dass die Aufnahmeeinrichtung von einem ersten Luftkanal gebildet ist, der vorzugsweise deckennah in dem Raum angeordnet ist und einen Abluftkanal einer Lüftungsanlage bildet und die Rückführeinrichtung von einem zweiten Luftkanal gebildet ist, der vorzugsweise deckennah in dem Raum angeordnet ist und einen Zuluftkanal der Lüftungsanlage bildet, wobei die Aufnahmeeinrichtung und die Rückführeinrichtung zu der außerhalb des Raums angeordneten Adsorptionseinrichtung führen.

Des Weiteren kann es von Vorteil sein, wenn mittels einer Adsorptionseinrichtung und von einem Vorrat des Desinfektionsmittels aus, mehrere Räume mit dem Desinfektionsmittel beaufschlagbar sind und aus mehreren Räumen mit Desinfektionsmittel versetzte Luft desinfizierbar ist. Hierbei können die zugehörigen Luftkanäle derart schaltbar sein, dass die Räume dennoch unabhängig voneinander desinfizierbar sind oder nach Art einer Parallelschaltung gleichzeitig desinfizierbar sind.

Gemäß einer anderen Ausführungsform der erfindungsgemäßen Vorrichtung ist es vorgesehen, dass die Vorrichtung mobil, insbesondere verfahrbar, ist und dass ein Eintrittsquerschnitt der Aufnahmeeinrichtung in einem Abstand zu einem Austrittsquerschnitt der Rückführeinrichtung oder der Abgabeeinrichtung angeordnet ist. Eine derartige Vorrichtung kann so ausgebildet sein, dass sie mit der Aufnahmeeinrichtung und der Rückführeinrichtung oder der Abgabeeinrichtung an entsprechende Leitungen einer vorhandenen Lüftungsanlage des Raums angeschlossen werden kann. Hierzu ist es des Weiteren vorteilhaft, wenn die Aufnahmeeinrichtung und die Rückführeinrichtung über flexible und in ihrer Länge veränderlichen Anschlussleitungen verfügt. Eine solche mobile Vorrichtung kann somit zur Desinfektion von verschiedenen Räumen an verschiedenen Orten eingesetzt werden.

Für den Fall, dass die erfindungsgemäße Vorrichtung in Räumen eingesetzt werden soll, die über keine Lüftungsanlage verfügen oder deren Lüftungsanlage für einen Anschluss der Vorrichtung ungeeignet sind, kann die Vorrichtung auch so ausgebildet sein, dass sie in den Raum verfahren werden kann, wobei der Eintrittsquerschnitt der Aufnahmeeinrichtung und der Austrittsquerschnitt der Rückführeinrichtung oder der Abgabeeinrichtung derart angeordnet werden können, dass die Luft innerhalb des Raums in einem Kreislauf geführt wird. Abgabeeinrichtung, Aufnahmeeinrichtung und Rückführeinrichtung können dann als Rohrstutzen ausgeführt sein, die auf gegenüberliegenden Seiten der mobilen Vorrichtung angeordnet sind, um eine schnellere und bessere Durchmischung mit der Raumluft zu gewährleisten.

Insbesondere ist es von Vorteil, wenn die Vorrichtung eine Luftfördereinrichtung, insbesondere einen Ventilator, aufweist, mit der oder dem sowohl der Strom des Desinfektionsmittels in die Luft des Raums als auch der Strom aus Luft und Desinfektionsmittel aus dem Raum in die Adsorptionseinrichtung und anschließend wieder zurück in den Raum förderbar ist, wobei mittels schaltbarer Absperreinrichtungen mindestens zwei verschiedene Luftwege einstellbar sind. Dabei ist es ferner von Vorteil, wenn die Aufgabeeinrichtung und die Adsorptionseinrichtung parallel geschaltet sind, wobei wahlweise entweder die Aufgabeeinrichtung oder die Absorptionseinrichtung durchströmbar ist.

Umfasst die Vorrichtung ferner eine Heizeinrichtung, mittels der die Luft des Raums, vorzugsweise vor Beginn der Desinfektion, aufheizbar ist, kann einem Kondensieren des eingebrachten Desinfektionsmittels entgegengewirkt werden.

Schließlich ist es vorteilhaft, wenn eine dritte Leitung zur Führung der Luft des Raums durch die Vorrichtung vorgesehen ist, mittels der die Aufgabeeinrichtung und die Adsorptionseinrichtung umgehbar sind. Darüber hinaus kann in einer der Leitungen beziehungsweise in der gemeinsamen Leitung ein Elektroheizregister als Heizeinrichtung angeordnet sein, das die Luft des Raums in einem reinen Heizbetrieb erwärmen kann.

### Ausführungsbeispiel

Die Erfindung wird nachfolgend anhand eines Ausführungsbeispiels einer erfindungsgemäßen Vorrichtung zur Desinfektion eines Raums, die in den Figuren schematisch dargestellt ist, näher erläutert.

Es zeigt
- Fig. 1:: eine schematische Darstellung einer erfindungsgemäßen Vorrichtung im Heizbetrieb,
- Fig. 2:: die Vorrichtung aus Figur 1 in der Desinfektionsphase,
- Fig. 3:: die Vorrichtung aus Figur 1 in der Adsorptionsphase,
- Fig. 4:: die Vorrichtung aus Figur 1 nach der Abkopplung von dem Lüftungssystem,
- Fig. 5:: die Vorrichtung aus Figur 1, angekoppelt vor dem Zuluftfilter und hinter der 2-stufigen Abluft-Filterung und
- Fig. 6:: eine schematische Darstellung einer alternativen Vorrichtung.

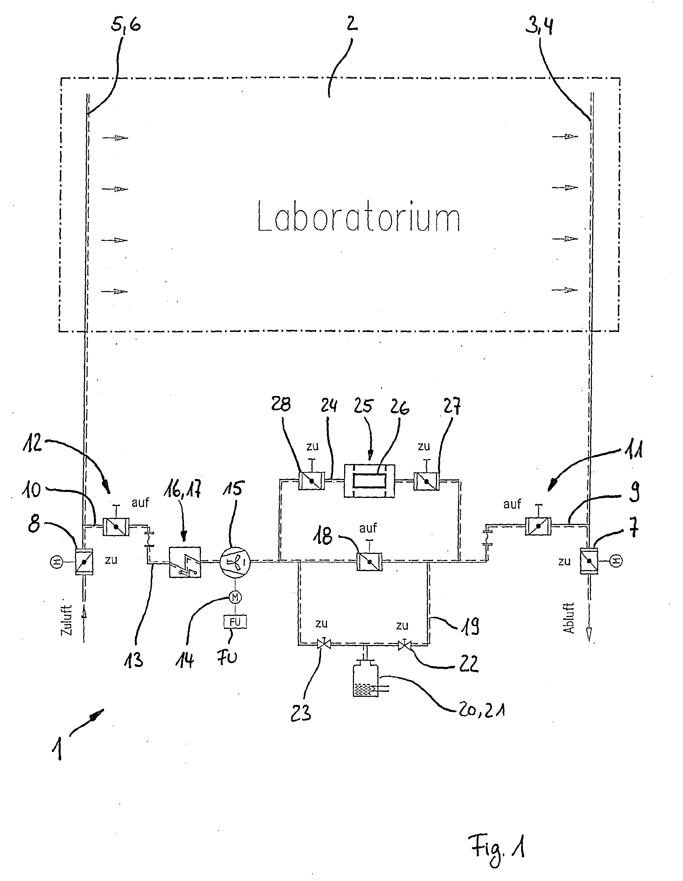

In der Figur 1 ist eine erfindungsgemäße Vorrichtung 1 zur Desinfektion eines Raums 2 schematisch dargestellt, wobei der Raum 1 ein Hochsicherheitslabor ist, das über eine in der Figur nur teilweise dargestellte Lüftungsanlage verfügt. Die Lüftungsanlage umfasst einen ersten Luftkanal 3, der deckennah in dem Raum 2 angeordnet ist und der einen Abluftkanal 4 der Lüftungsanlage bildet. Des weiteren weist die Lüftungsanlage einen zweiten Luftkanal 5 auf, der einen Zuluftkanal 6 bildet, wobei dieser ebenfalls deckennah in dem Raum 2 angeordnet ist. Sowohl der Abluftkanal 4 als auch der Zuluftkanal 6 verfügen über eine gasdichte Absperrklappe 7, 8, die sich jeweils außerhalb des Raums 2 befindet und über die die beiden Kanäle 4 und 6 gasdicht verschlossen werden können. Sind beide Absperrklappen 7, 8 geschlossen - wie es in der Figur 1 der Fall ist, so ist der Raum 2 des Labors zu der übrigen Lüftungsanlage hin hermetisch verriegelt.

Jeweils im Bereich zwischen dem Raum 2 und den Absperrklappen 7, 8 sind die beiden Kanäle 4 und 6 jeweils mit einem Anschlussstutzen 9, 10 versehen, wobei die beiden Anschlussstutzen 9, 10, von denen jeweils eine Abzweigleitung ausgeht, abermals mit jeweils einer gasdichten Absperrklappe 11, 12 versehen sind.

Die erfindungsgemäße Vorrichtung 1 ist zwischen die beiden Absperrklappen 11 und 12 geschaltet, die in der Figur 1 jeweils geöffnet sind. Die Vorrichtung 1 weist eine Hauptleitung 13 auf, in der ein über einen Motor 14 betriebener Ventilator 15 und eine als Elektroheizregister 16 ausgebildete Heizeinrichtung 17 angeordnet ist, wobei der Motor 14 des Ventilators 15 mittels eines Frequenzumrichters FU in seiner Drehzahl, das heißt in seinem Volumenstrom, angepasst werden kann und im vorliegenden Fall auf ca. 1000 m³/h eingestellt ist. Innerhalb der Hauptleitung 13 ist eine weitere gasdichte Absperrklappe 18 vorgesehen, mittels derer die Hauptleitung 13 unterbrochen werden kann. Die Hauptleitung 13 ist an ihren beiden Enden jeweils mit einem flexiblen Leitungsstück an die Anschlussstutzen 9, 10 angeschlossen.

Über eine erste Bypass-Leitung 19 kann der Hauptleitung 13 eine Abgabeeinrichtung 20 für ein Desinfektionsmittel, nämlich gemäß Figur 1 ein Formalin-Verdampfer 21, zugeschaltet werden, wobei die erste Bypass-Leitung 19 mit zwei Kugelhähnen 22, 23 ausgestattet ist, mittels derer die erste Bypass-Leitung 19 vor und hinter dem Formalin-Verdampfer 21 abgesperrt und letztgenannter gefahrlos entfernt werden kann.

Darüber hinaus verfügt die Hauptleitung 13 über eine zweite Bypass-Leitung 24, in der eine Adsorptionseinrichtung 25 mit einem entsprechenden Vorrat 26 an Adsorptionsmittel, nämlich Aktivkohle, angeordnet ist. Die zweite Bypass-Leitung 24 ist abermals vor und hinter der Adsorptionseinrichtung 25 mit jeweils einer gasdichten Absperrklappe 27, 28 versehen.

Die Hauptleitung 13 und die zwei Bypass-Leitungen 19, 24 sind parallel geschaltet, wobei durch die Stellung der einzelnen gasdichten Absperrklappen 11, 12, 18, 27, 28 sowie der Kugelhähne 22, 23 die jeweilige Funktion bestimmt wird, die die erfindungsgemäße Vorrichtung 1 ausübt. In Figur 1, die die Vorrichtung 1 im Heizbetrieb zeigt, sind die Absperrklappen 7, 8 der Lüftungsanlage des Raums 2 geschlossen, während die Absperrklappen 11, 12, 18 geöffnet sind und der Ventilator 15 eingeschaltet ist, so dass die Luft des Raums 2 in die Vorrichtung 1 gesaugt wird. Die Kugelhähne 22, 23 und die Absperrklappen 27, 28 der beiden Bypass-Leitungen 19, 24 sind geschlossen, so dass die von dem Ventilator 15 angesaugte Luft lediglich durch die Hauptleitung 13 geführt wird, in der sich der ebenfalls in Betrieb befindliche Elektroheizregister 16 befindet. Die aus dem Raum 2 angesaugte Luft wird mittels des Elektroheizregisters 16 aufgewärmt und wieder in den Raum 2 abgegeben, so dass sich die Lufttemperatur innerhalb des Raums 2 sowie die Oberflächen der in dem Raum 2 befindlichen Teile auf ca. 30°C erwärmen. Auf diese Weise wird eine Kondensation des Formaldehyds in der folgenden Desinfektionsphase vermieden.

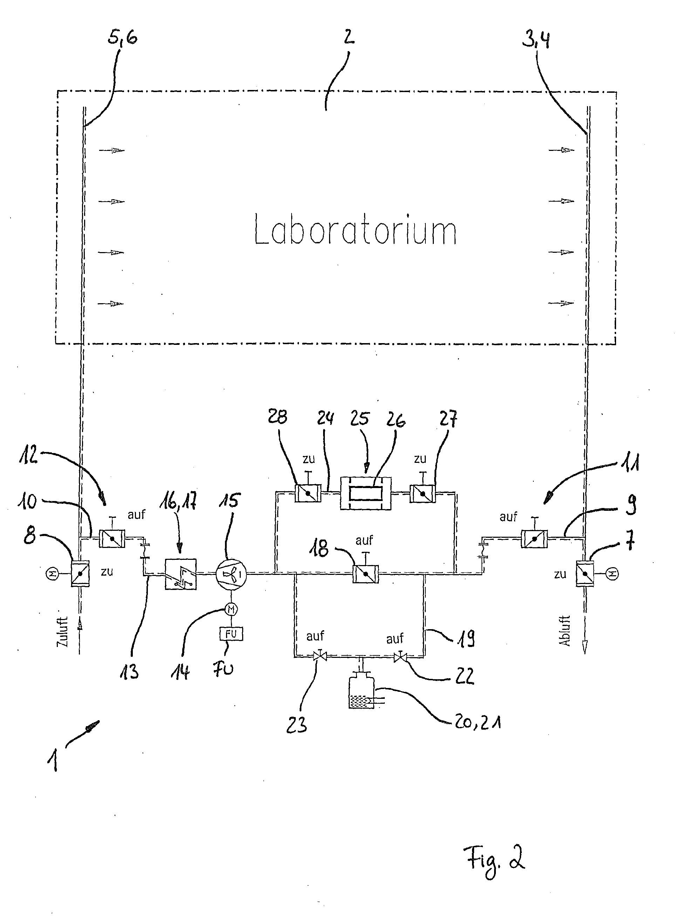

Die Figur 2 zeigt die erfindungsgemäße Vorrichtung 1 aus Figur 1, wobei die einzelnen Absperrklappen 11, 12, 18, 27, 28 und Kugelhähne 22, 23 entsprechend der Desinfektionsphase der Vorrichtung 1 eingestellt sind. Die Absperrklappen 7, 8 der Lüftungsanlage sowie die Absperrklappen 27, 28 der zweiten Bypass-Leitung 24 bleiben geschlossen. Ferner bleibt die Absperrklappe 18 der Hauptleitung 13 geöffnet und die Kugelhähne 22, 23 der ersten Bypass-Leitung 19 werden geöffnet, so dass die durch den Ventilator 15 angesaugte Luft mit dem von dem Formalin-Verdampfer 21 abgegebenen Desinfektionsmittel versetzt und wieder in den Raum 2 eingeleitet wird. Das Elektroheizregister 16 bleibt auch während der Desinfektionsphase weiterhin in Betrieb, um einem Abkühlen der Luft- und Oberflächentemperaturen in dem Raum 2 entgegen zu wirken.

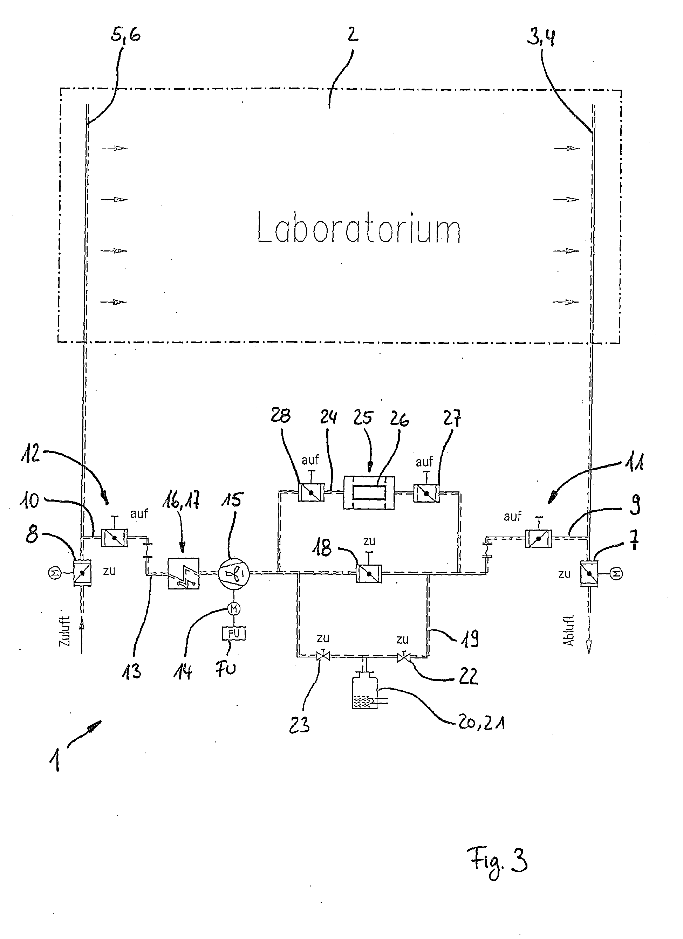

Nach Ablauf der Einwirkzeit des Desinfektionsmittels werden die Kugelhähne 22, 23 geschlossen und die Absperrklappen 27, 28 geöffnet, so dass die Luft des Raums 2 durch die Adsorptionseinrichtung 25 geführt wird und das Desinfektionsmittel an dem Adsorptionsmittel adsorbiert wird. Die entsprechende Stellung der Absperreinrichtungen ist in der Figur 3 dargestellt.

Die Luft des Raums 2 wird solange durch die Adsorptionseinrichtung 25 geleitet, bis die Konzentration des Desinfektionsmittels in der Luft des Raums unter einen Grenzwert abgesenkt worden ist. Eine Messung der aktuellen Konzentration des Desinfektionsmittels in der Luft kann mit geeigneten Mitteln zum Beispiel in dem Abluftkanal 4, der zu der Adsorptionseinrichtung 25 führt, durchgeführt werden. Auch während der hier beschriebenen Adsorptionsphase bleibt das Elektroheizregister 16 in Betrieb.

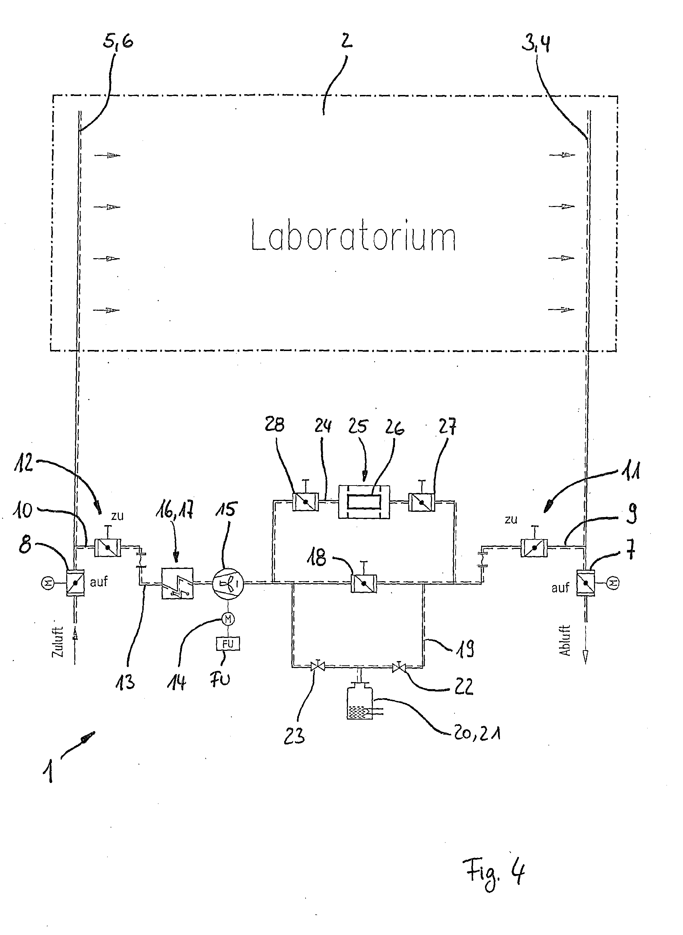

Nach beendeter Desinfektion des Raums 2 kann die Vorrichtung 1 von der Lüftungsanlage abgekoppelt werden. Hierzu ist es lediglich erforderlich, die beiden gasdichten Absperrklappen 11, 12 zu schließen und die flexiblen Leitungen der Vorrichtung 1 an den beiden Enden der Hauptleitung 13 von den entsprechenden Anschlussstutzen 9, 10 zu trennen. Diese Situation ist schematisch in Figur 4 gezeigt. Zur Wiederaufnahme des normalen Lüftungsbetriebes werden die Absperrklappen 7 und 8 wieder geöffnet.

Die Vorrichtung 1 kann fest installiert sein oder als mobile Einheit ausgebildet sein und somit nacheinander in der Nähe verschiedener Räume eines Gebäudes platziert und betrieben werden.

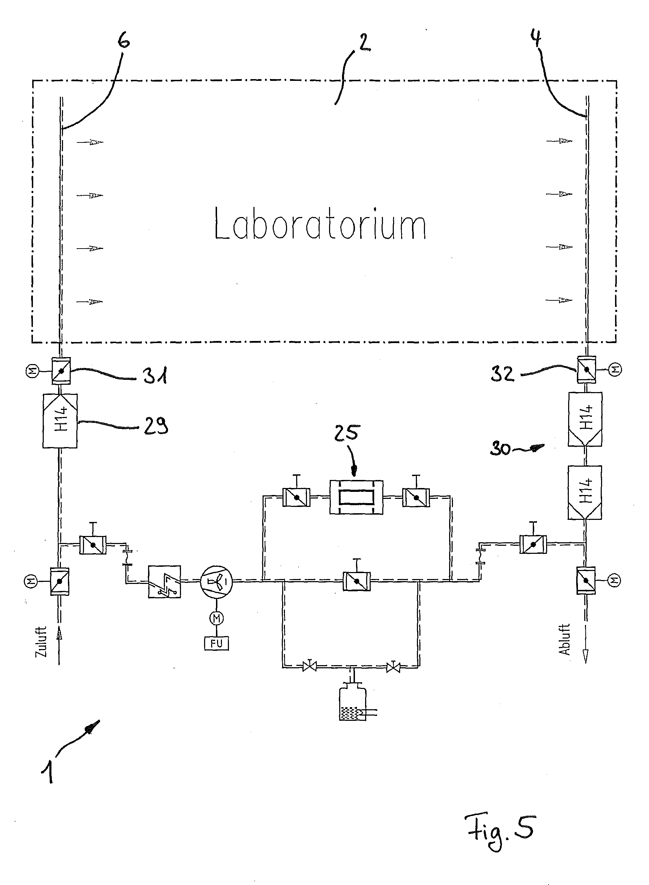

In der Figur 5 ist abermals die Vorrichtung1 aus Figur 1 gezeigt, die zwischen dem Zuluftkanal 6 und dem Abluftkanal 4 angeordnet ist. Im Unterschied zu Figur 1 ist gemäß Figur 5 im Zuluftkanal 6 ein Zuluftfilter 29 und im Abluftkanal 4 eine zweistufige Abluftfilterung 30 angeordnet. Sowohl Zuluftkanal 6 als auch Abluftkanal 4 können über jeweils eine zusätzliche Absperrklappe 31, 32 verschlossen werden.

Demnach erfolgt die Ankopplung der Vorrichtung 1 in Srtömungsrichtung vor dem Zuluftfilter 29 und hinter der Abluftfilterung 30. Dies ist insbesondere vorteilhaft, da gleichzeitig das Zuluftfilter 29 einschließlich des Zuluftkanals 6, der Raum 2 und die Abluftfilterung 30 desinfiziert wird. Nach Ablauf der Einwirkzeit wird die mit dem Desinfektionsmittel versetzte Luft dann über die Adsorptionseinrichtung 25 geführt, wodurch der Formalingehalt auf den zulässigen Wert reduziert wird.

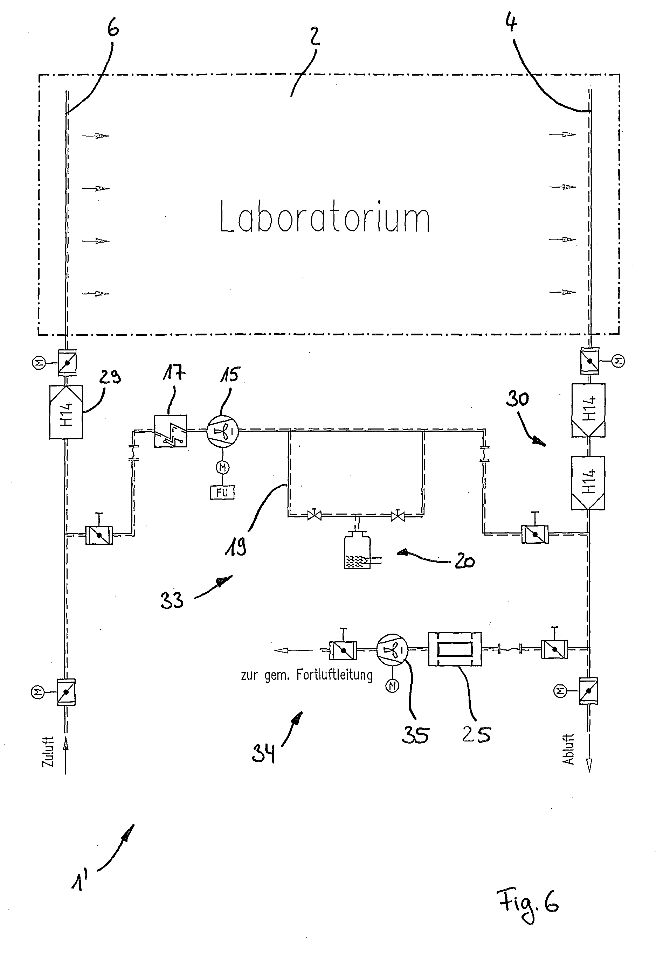

In der Figur 6 ist eine alternative Ausbildung einer erfindungsgemäßen Vorrichtung 1' dargestellt, bei der die Desinfektionsphase und die Adsorptionsphase mittels zwei voneinander getrennten Einheiten 33, 34 erfolgen. Die erste Einheit 33 besteht aus einem Leitungssystem, innerhalb dem der Ventilator 15, die Heizeinrichtung 17 und die Abgabeeinrichtung 20 für das Desinfektionsmittel angeordnet sind, wobei die Abgabeeinrichtung 20 abermals in einer Bypass-Leitung 19 angeordnet ist, so dass mittels der Einheit 33 auch ein separater Heizbetrieb erfolgen kann. Die Einheit 33 ist als mobile Einheit ausgebildet und kann je nach Bedarf an die Lüftungsanlage verschiedener Räume angeschlossen werden. Darüber hinaus kann die Einheit 33 auch in einen Raum 2 verfahren und dort betrieben werden, ohne an eine Lüftungsanlage angeschlossen zu sein. Da die einzelnen Bestandteile der Einheit 33 jeweils relativ klein sind, ist die Einheit 33 sehr kompakt und handlich und erlaubt einen flexiblen Einsatz.

Bei der Einheit 34 handelt es sich um eine stationäre Einrichtung, die einen Ventilator 35 und die Adsorptionseinrichtung 25 umfasst. Die Einheit 34 ist mit einer zentralen Lüftungsanlage verbunden und somit Teil derselben. Unabhängig von dem Einsatzort der Einheit 33 zur Aufgabe des Desinfektionsmittels wird also immer die zentrale Einheit 34 zur Adsorption des Desinfektionsmittels herangezogen, wobei die verschiedenen Lüftungsleitungen mit entsprechenden Absperrklappen bedarfsweise geöffnet und geschlossen werden können.

### Bezugszeichenliste:

- 1: Vorrichtung
- 2: Raum
- 3: Erster Luftkanal
- 4: Abluftkanal
- 5: Zweiter Luftkanal
- 6: Zuluftkanal
- 7: gasdichte Absperrklappe
- 8: gasdichte Absperrklappe
- 9: Anschlussstutzen
- 10: Anschlussstutzen
- 11: gasdichten Absperrklappe
- 12: gasdichten Absperrklappe
- 13: Hauptleitung
- 14: Motor
- 15: Ventilator
- 16: Elektroheizregister
- 17: Heizeinrichtung
- 18: Gasdichte Absperrklappe
- 19: Erste Bypass-Leitung
- 20: Abgabeeinrichtung
- 21: Formalin-Verdampfer
- 22: Kugelhahn
- 23: Kugelhahn
- 24: Zweite Bypass-Leitung
- 25: Adsorptionseinrichtung
- 26: Vorrat
- 27: gasdichten Absperrklappe
- 28: gasdichten Absperrklappe
- 29: Zuluftfilter
- 30: Abluftfilterung
- 31: Absperrklappe
- 32: Absperrklappe
- 33: Einheit
- 34: Einheit
- 35: Ventilator
- FU: Frequenzumrichter

## Patentansprüche

1. Verfahren zur Desinfektion eines Raums (2), insbesondere zum Abtöten von pathogenen Mikroorganismen wie Bakterien, Viren, Pilzen und deren Sporen, wobei der Raum (2) vor Beginn des Desinfektionsvorgangs gasdicht abgeschlossen wird und in den Raum (2) ein Desinfektionsmittel eingebracht wird und im Wesentlichen an allen Orten in dem Raum (2) eine für die beabsichtigte Desinfektionswirkung hinreichend hohe Konzentration des Desinfektionsmittels in der Luft des Raums (2) erzeugt wird und wobei das Desinfektionsmittel während einer Zeitdauer auf die in dem Raum (2) befindlichen Mikroorganismen einwirkt, **dadurch gekennzeichnet, dass** die Luft des Raums (2) nach Ablauf der Zeitdauer so lange über oder durch ein Adsorptionsmaterial geführt und das Desinfektionsmittel an dem Adsorptionsmaterial adsorbiert wird, bis die Konzentration des Desinfektionsmittels in der Luft des Raums (2) unter einen Grenzwert abgesenkt worden ist.

2. Verfahren nach Anspruch 1, **dadurch gekennzeichnet, dass** das Desinfektionsmittel Formaldehyd oder Formalin und das Adsorptionsmaterial Aktivkohle ist.

3. Verfahren nach Anspruch 1 oder 2, **dadurch gekennzeichnet, dass** die Luft innerhalb des Raums (2) in einem Kreislauf und dabei über oder durch das Adsorptionsmaterial geführt wird, wobei die Luft an einer ersten Stelle in dem Raum (2) angesaugt und an einer zweiten Stelle in dem Raum (2) wieder abgegeben wird.

4. Verfahren nach einem der Ansprüche 1 bis 3, **dadurch gekennzeichnet, dass** die Luft innerhalb der Raums (2) vor und/oder während der Einbringung des Desinfektionsmittels erwärmt wird.

5. Vorrichtung (1) zur Desinfektion eines Raums (2), insbesondere zum Abtöten von pathogenen Mikroorganismen wie Bakterien, Milben, Pilzen und deren Sporen, mit einem Vorrat eines Desinfektionsmittels und einer Abgabeeinrichtung, von der ein Strom des Desinfektionsmittels in die Luft des Raums (2) abgebbar ist, **gekennzeichnet durch** eine Aufnahmeeinrichtung, von der ein Strom aus Luft und Desinfektionsmittel aus dem Raum (2) in die Vorrichtung (1) aufnehmbar ist und eine Adsorptionseinrichtung (25), die einen Vorrat (26) eines Adsorptionsmaterials enthält, über oder **durch** das der von der Aufnahmeeinrichtung aufgenommene Strom leitbar ist, wodurch das Desinfektionsmittel aus dem Strom entfernbar und somit die Konzentration des Desinfektionsmittels in der Luft in dem Raum (2) absenkbar ist.

6. Vorrichtung (1) nach Anspruch 5, **dadurch gekennzeichnet, dass** der über oder durch das Adsorptionsmaterial geleitete und in seiner Konzentration des Desinfektionsmittels reduzierte Strom von einer Rückführeinrichtung, die zumindest teilweise von der Abgabeeinrichtung gebildet ist, in den Raum (2) zurückführbar ist.

7. Vorrichtung (1) nach Anspruch 5 oder 6, **dadurch gekennzeichnet, dass** die Vorrichtung (1) fest in einem Gebäude, das den Raum (2) aufweist, installiert ist und dass die Aufnahmeeinrichtung von einem ersten Luftkanal (3) gebildet ist, der vorzugsweise deckennah in dem Raum (2) angeordnet ist und einen Abluftkanal (4) einer Lüftungsanlage bildet und die Rückführeinrichtung von einem zweiten Luftkanal (5) gebildet ist, der vorzugsweise deckennah in dem Raum (2) angeordnet ist und einen Zuluftkanal (6) der Lüftungsanlage bildet, wobei die Aufnahmeeinrichtung und die Rückführeinrichtung zu der außerhalb des Raums (2) angeordneten Adsorptionseinrichtung (25) führen.

8. Vorrichtung nach Anspruch 7, **dadurch gekennzeichnet, dass** mittels einer Adsorptionseinrichtung und von einem Vorrat des Desinfektionsmittels aus, mehrere Räume mit dem Desinfektionsmittel beaufschlagbar sind und aus mehreren Räumen mit Desinfektionsmittel versetzte Luft desinfizierbar ist.

9. Vorrichtung (1) nach Anspruch 6 oder 7, **dadurch gekennzeichnet, dass** die Vorrichtung (1) mobil, insbesondere verfahrbar, ist und dass ein Eintrittsquerschnitt der Aufnahmeeinrichtung in einem Abstand zu einem Austrittsquerschnitt der Rückführeinrichtung oder der Abgabeeinrichtung angeordnet ist.

10. Vorrichtung (1) nach einem der Ansprüche 5 bis 9, **gekennzeichnet durch** eine Luftfördereinrichtung, insbesondere einen Ventilator (15), mit der oder dem sowohl der Strom des Desinfektionsmittels in die Luft des Raums (2) als auch der Strom aus Luft und Desinfektionsmittel aus dem Raum (2) in die Adsorptionseinrichtung (25) und anschließend wieder zurück in den Raum (2) förderbar ist, wobei mittels schaltbarer Absperreinrichtungen mindestens zwei verschiedene Luftwege einstellbar sind.

11. Vorrichtung (1) nach einem der Ansprüche 5 bis 10, **dadurch gekennzeichnet, dass** die Aufgabeeinrichtung und die Adsorptionseinrichtung (25) parallel geschaltet sind, wobei wahlweise entweder die Aufgabeeinrichtung oder die Absorptionseinrichtung (25) durchströmbar ist.

12. Vorrichtung (1) nach einem der Ansprüche 5 bis 11, **gekennzeichnet durch** eine Heizeinrichtung (17), mittels der die Luft des Raums (2) aufheizbar ist.

13. Vorrichtung nach einem der Ansprüche 5 bis 12, **gekennzeichnet durch** eine dritte Leitung zur Führung der Luft des Raums (2) **durch** die Vorrichtung (1), mittels der die Aufgabeeinrichtung und die Adsorptionseinrichtung (25) umgehbar sind.
